# EUROPÄISCHE PATENTSCHRIFT

(11) **EP 4 346 736 B1**
(45) Veröffentlichungstag und Bekanntmachung des Hinweises auf die Patenterteilung: **05.11.2025**
(21) Anmeldenummer: 22800140.0
(22) Anmeldetag: 30.09.2022
(51) Int. Cl.: A61J 1/10, A01N 1/00, B01J 20/00, B32B 1/00

(54) **TRANSPORTBEUTEL ZUM TRANSPORTIEREN VON FLÜSSIGKEITSPROBEN UND VERFAHREN ZU SEINER HERSTELLUNG**
TRANSPORT BAG FOR TRANSPORTING LIQUID SAMPLES AND METHOD FOR THE PRODUCTION THEREOF
SAC DE TRANSPORT POUR ASSURER LE TRANSPORT D'ÉCHANTILLONS LIQUIDES ET SON PROCÉDÉ DE FABRICATION

(30) Priorität: 07.10.2021 DE 102021125990
(43) Veröffentlichungstag der Anmeldung: 10.04.2024
(73) Patentinhaber: Anton Debatin GmbH Werk für werbende Verpackung, 76646 Bruchsal (DE)
(72) Erfinder: ROSE, Thomas, 34346 Hann.Münden (DE); TRÖBER, Oliver, 75015 Bretten (DE)
(74) Vertreter: Lichti - Patentanwälte Partnerschaft mbB
(86) Internationale Anmeldenummer: PCT/EP2022/077350
(87) Internationale Veröffentlichungsnummer: WO 2023/057342

(56) Entgegenhaltungen:
- WO-A1-97/11009
- DE-A1- 10 301 171
- JP-A- H11 128 315
- US-A- 4 861 632
- US-A- 5 706 937

## Beschreibung

Die Erfindung betrifft, wie in Anspruch 7 dargelegt, einen Transportbeutel zum Transportieren von Flüssigkeitsproben, insbesondere Blut-, Urin-, Gewebeproben oder dergleichen, mit einer 1. Folienlage und einer 2. Folienlage, die jeweils aus Kunststoff bestehen und unter Bildung eines zwischen den Folienlagen ausgebildeten Innenraums aufeinanderliegend angeordnet und miteinander verbunden sind, wobei im Innenraum eine Absorbereinrichtung zum Aufnehmen von Flüssigkeit angeordnet ist, die Absorberpartikel aufweist, die an und/oder in einem Trägermaterial angeordnet sind, wobei das Trägermaterial auf der dem Innenraum zugewandten Innenseite zumindest einer der Folienlagen gehalten ist.

Desweiteren betrifft die Erfindung, wie in Anspruch 1 dargelegt, ein Verfahren zur Herstellung eines entsprechenden Transportbeutels, wobei zwei Folienlagen aus Kunststoff aufeinander gelegt und miteinander so verbunden werden, dass zwischen den Folienlagen ein Innenraum gebildet ist.

Ein Transportbeutel der genannten Art ist beispielsweise in der US 4861632 A, weiterhin in der DE 202012100517 U1 beschrieben. Beim Transportieren von Flüssigkeitsproben besteht die Gefahr, dass ein die Flüssigkeitsprobe aufnehmendes Probegefäß entweder undicht oder beschädigt wird, so dass die Probe innerhalb des Transportbeutels ausläuft. Um zu verhindern, dass die Flüssigkeitsprobe an die Umgebung gelangt, ist es bekannt, das Probengefäß vor dem Einsetzen in den Transportbeutel mit einem saugfähigen Material, beispielsweise einem Vlies-Zuschnitt zu umhüllen. Alternativ oder zusätzlich dazu kann auch ein saugfähiges Element, beispielsweise ein Vlies-Zuschnitt in den Innenraum des Transportbeutels eingelegt werden, wobei zusätzlich Partikel aus SAP (superabsobierendes Polymer) zugesetzt sein können. Das vorgefertigte saugfähige Element kann mit der Innenwandung zumindest einer der Folienlagen verklebt werden. In allen Fällen ist die Vorfertigung, die Anbringung oder das Einsetzen des saugfähigen Elementes mühsam und zeitaufwendig, wodurch die Herstellung des Transportbeutels kostenintensiv ist.

Der Erfindung liegt die Aufgabe zu Grunde, ein Verfahren zur Herstellung eines entsprechenden Transportbeutels zu schaffen, das eine automatisierte Herstellung des Transportbeutels ermöglicht
Darüber hinaus soll ein Transportbeutel geschaffen werden, der sich in einfacher Weise herstellen lässt.

Hinsichtlich des Verfahrens wird die oben genannte Aufgabe erfindungsgemäß dadurch gelöst, dass vor dem Aufeinanderlegen der beiden Folienlagen auf zumindest eine der Folienlagen ein flüssiges oder zähflüssiges Trägermaterial aufgebracht wird, in das Absorberpartikel eingemischt sind oder eingemischt werden. Die Aufbringung des Trägermaterials kann durch beispielsweise Sprühen, Spritzen, Drucken, Rakeln oder Streichen erfolgen. Anschließend wird das Trägermaterial mit den darin enthaltenen Absorberpartikeln getrocknet und/oder gekühlt. Die Trocknung und/oder die Kühlung kann an der Umgebungsluft und/oder durch Zuführung und insbesondere Aufblasen von Warmluft zur Trocknung oder Kaltluft zur Kühlung geschehen. Vorzugsweise erfolgt die Trocknung und/oder Kühlung, bevor die Folienlagen aufeinandergelegt werden.

In bevorzugter Ausgestaltung der Erfindung ist vorgesehen, dass ein aushärtbarer Kunststoff und/oder Klebstoff als das Trägermaterial verwendet wird. Damit ist der Vorteil verbunden, dass die Absorberpartikel in den noch flüssigen oder zähflüssigen Kunststoff und/oder Klebstoff eingemischt und/oder eingebettet werden können, woraufhin der Kunststoff und/oder Klebstoff auf zumindest eine der Folienlagen aufgetragen werden kann und dort aushärtet und somit mit der entsprechenden Folienlage verbunden ist. Vorzugsweise sind die Absorberpartikel bereits in das Trägermaterial eingebracht, bevor dieses auf die Folienlage aufgebracht wird.

Als Trägermaterialien können beispielsweise Polyethylenoxide, Poly-N-vinylpyrrolidone, Polyvinylalkohole und Polyacrylamide oder eine Mischung dieser Materialen verwendet werden.

In Weiterbildung der Erfindung kann vorgesehen sein, dass solche Absorberpartikel verwendet werden, die zumindest überwiegend aus zumindest einem superabsorbierenden Polymer (SAP) bestehen. Ein entsprechendes Polymer ist in der Lage, ein Vielfaches seines Eigengewichtes an polaren Flüssigkeiten, insbesondere Wasser oder wässrigen Lösungen aufzusaugen.

Bei dem superabsorbierenden Polymer kann es sich beispielsweise um ein Copolymer aus Acrylsäure oder Natriumacrylat einerseits und Acrylamit andererseits handeln.

Die Absorberpartikel werden vorzugsweise als Granulat mit einer Partikelgröße im Bereich von 100µm bis 1000µm verwendet.

In bevorzugter Ausgestaltung der Erfindung kann vorgesehen sein, dass die Absorberpartikel in einem Anteil von 20mas% bis 80mas% der Gesamtmasse aus Trägermaterial und Absorberpartikeln vorhanden sind. Insbesondere ist für die Absorberpartikel ein Anteil von 30mas% bis 50mas% an der Gesamtmasse aus Trägermaterial und Absorberpartikeln vorgesehen.

Das Trägermaterial kann mit der es haltenden Folienlage unlösbar verbunden und insbesondere verschweißt oder stoffschlüssig verbunden sein. In bevorzugter Ausgestaltung der Erfindung ist jedoch vorgesehen, dass das Trägermaterial abziehbar an der Folienlage haften kann, so dass auch nach Gebrauch des Transportbeutels eine Trennung des Trägermaterials von der Folienlage des Transportbeutels möglich ist.

Das Trägermaterial ist beispielsweise streifenförmig ausgebildet und vorzugsweise in demjenigen Endbereich des Transportbeutels angeordnet, der einer Einführöffnung, durch die der Innenraum des Transportbeutels zugänglich ist, abgewandt ist. Als Endbereich wird das untere Drittel oder das untere Viertel der Länge des Transportbeutels angesehen.

Der Transportbeutel ist vorzugsweise an seinen seitlichen Rändern mittels einer die beiden Folienlagen verbindenden Schweißnaht jeweils verbunden. Es ist vorgesehen dass sich das Trägermaterial im Wesentlichen parallel und im Abstand zu zumindest einer der Schweißnähte erstreckt.

In einer weiteren alternativen Ausgestaltung kann vorgesehen sein, dass zwei streifenförmig ausgebildete Trägermaterialien der genannten Art vorhanden sind, in die jeweils die Absorberpartikel eingemischt und/oder eingebettet sind. In einer möglichen Ausgestaltung des Verfahrens ist vorgesehen, dass die beiden Folienlagen von einer einstückigen Folie gebildet werden, die unter Bildung einer Falzkante so umgefalzt wird, dass die beiden aufeinanderliegenden Folienlagen gebildet sind. Vor dem Umlegen der Folienlagen wird auf die Folie das Trägermaterial insbesondere ein Form eines aushärtbaren Kunststoffs und/oder Klebstoffs in genannter Weise aufgebracht. Bereits vor dem Aufbringen des Kunststoffs und/oder Klebstoffs können die Absorberpartikel in den Kunststoff und/oder Klebstoff eingemischt sein. Alternativ oder zusätzlich dazu kann vorgesehen sein, dass die Absorberpartikel nach dem Aufbringen des Kunststoffs und/oder Klebstoffs auf die Folie auf dem Kunststoff und/oder Klebstoff aufgebracht und/oder in diesen eingebettet werden.

Nachdem das Trägermaterial beziehungsweise der Kunststoff und/oder Klebstoff gekühlt und/oder getrocknet oder zumindest angetrocknet ist, wird die Folie unter Bildung der beiden aufeinanderliegenden Folienlagen umgelegt beziehungsweise umgefalzt und die Folienlagen werden durch Ausbildung von zwei auf Abstand angeordneten Schweißnähten miteinander verbunden. Die Zugangsöffnung kann zwischen den Folienlagen vorgesehen sein. Alternativ ist es möglich, eine der Folienlagen zu schlitzen, wobei der so gebildete Schlitz die Zugangsöffnung bildet.

In oder an der Zugangsöffnung kann ein Sicherheitsverschluss ausgebildet sein, wie er bei Sicherheits-Transportbeuteln üblich ist. Ein entsprechender Sicherheitsverschluss ist so ausgebildet, dass es von außen erkennbar ist, wenn nach dem Verschließen des Beutels versucht wurde, diesen beispielsweise durch Einwirkung von Wärme, Kälte oder Chemikalien zu öffnen.

Zur Lösung der Aufgabe in vorrichtungstechnischer Hinsicht ist ein Transportbeutel vorgesehen, wobei eine Absorbereinrichtung zum Aufnehmen von Flüssigkeit Absorberpartikel aufweist, die an und/oder in einem Trägermaterial angeordnet sind, wobei das Trägermaterial auf der dem Innenraum zugewandten Innenseite zumindest einer der Folienlagen gehalten ist. Als Trägermaterial ist ein ausgehärteter Kunststoff und/oder Klebstoff vorgesehen, der zusammen mit den Absorberpartikeln auf die Folie in genannter Weise aufgebracht und insbesondere aufgespritzt oder aufgedruckt sein kann. Ausgestaltungen und Weiterbildungen des Transportbeutels ergeben sich aus den vorstehenden Erläuterungen zu dem Verfahren.

Weitere Einzelheiten und Merkmale der Erfindung sind aus der folgenden Beschreibung eines Ausführungsbeispiels unter Bezugnahme auf die Zeichnungen ersichtlich. Es zeigen:
- Fig. 1: eine Vorderansicht eines erfindungsgemäßen Transportbeutels zum Transportieren von Flüssigkeitsproben und
- Fig. 2: den Schnitt II-II in Fig. 1.

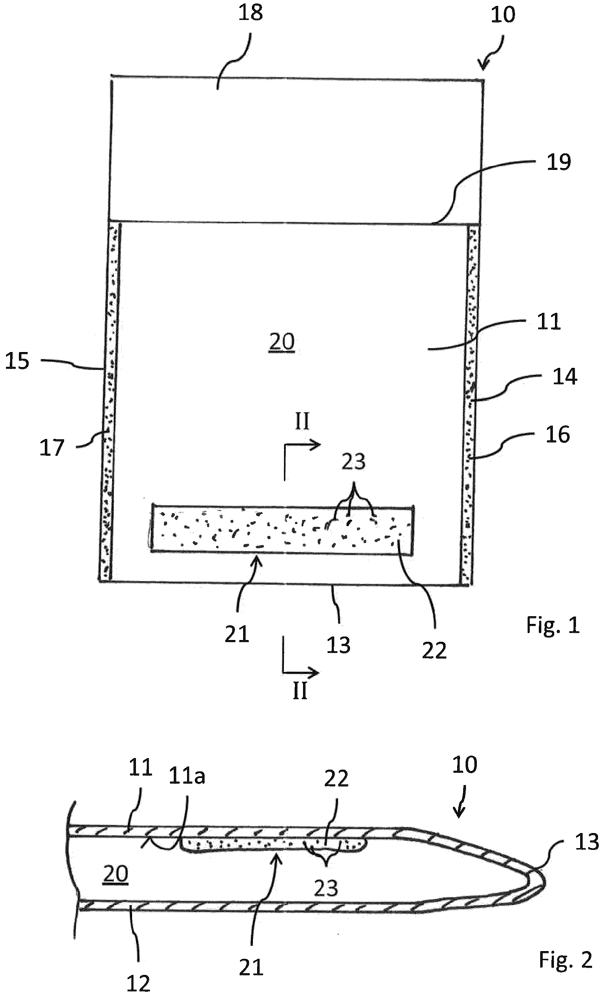

Ein in den Figuren 1 und 2 dargestellter Transportbeutel 10 aus Kunststoff zum Transportieren von Flüssigkeitsproben besitzt eine 1. Folienlage 11 und eine 2. Folienlage 12, die über- und aufeinanderliegend angeordnet sind. Die beiden Folienlagen 11 und 12 sind vorzugsweise dadurch gebildet, dass eine Kunststofffolie unter Bildung einer unteren Falzkante 13 umgeschlagen und aufeinandergelegt ist.

Die untere 2. Folienlage 12 steht gemäß Fig. 1 auf der der Falzkante 13 abgewandten Oberseite des Transportbeutels 10 über die obere 1. Folienlage 11 unter Bildung einer Lasche 18 hervor.

Die beiden Folienlagen 11 und 12 sind durch seitliche Schweißnähte 16 und 17 an ihren Seitenrändern 14 und 15 miteinander verbunden, so dass zwischen den beiden Folienlagen 11 und 12 ein Innenraum 20 gebildet ist, der nur durch eine zwischen den beiden Folienlagen 11 und 12 im Bereich der Lasche 18 gebildete Zugangsöffnung 19 zugänglich ist. Die Zugangsöffnung 19 kann mittels einer nicht dargestellten Verschlussvorrichtung verschlossen werden, bei der es sich beispielsweise auch um einen sogenannten Originalitätsverschluss oder einen Sicherheitsverschluss handeln kann, der eine nachträgliche Manipulation an dem verschlossenen Transportbeutel 10 anzeigt.

Im Innenraum 20 des Transportbeutels 10 ist eine Absorbereinrichtung 21 angeordnet, mittels der eine im Innenraum 20 befindliche Flüssigkeit aufgesaugt und absorbiert werden kann. Die Absorbereinrichtung 21 umfasst ein Trägermaterial 22 in Form eines aushärtbaren oder ausgehärteten Kunststoffs und/oder Klebstoffs, an und/oder in dem Absorberpartikel 23 gehalten sind. Die Absorberpartikel 23 können in das Trägermaterial 22 eingemischt und/oder auf dieses beispielsweise aufgestreut sein.

Die Absorbereinrichtung 21 ist bei dem dargestellten Ausführungsbeispiel auf der dem Innenraum 20 zugwandten Innenseite 11a der oberen 1. Folienlage 11 angeordnet und an der 1. Folienlage 11 gehalten. Dabei kann die Absorbereinrichtung 21 mit der 1. Folienlage 11 verklebt oder stoffschlüssig verbunden sein, wobei die Absorbereinrichtung 21 gegebenenfalls an der 1. Folienlage 11 nur in lösbarer Weise anhaftet und nachträglich wieder abgezogen werden kann.

## Patentansprüche

1. Verfahren zur Herstellung eines Transportbeutels zum Transportieren von Flüssigkeitsproben, insbesondere Blut-, Urin-, Gewebeproben oder dergleichen, wobei zwei Folienlagen (11, 12) aus Kunststoff aufeinander gelegt und unter Bildung einer Schweißnaht (16) miteinander so verschweißt werden, dass zwischen den Folienlagen (11, 12) ein Innenraum (20) gebildet ist, wobei vor dem Aufeinanderlegen der Folienlagen (11, 12) auf zumindest eine der Folienlagen (11, 12) ein flüssiges oder zähflüssiges Trägermaterial (22) aufgebracht wird, in das Absorberpartikel (23) eingemischt sind oder eingemischt werden, und dass das Trägermaterial (22) mit den Absorberpartikeln (23) anschließend getrocknet und/oder gekühlt wird, wobei das Trägermaterial (22) so aufgesprüht oder aufgespritzt oder aufgedruckt oder aufgerakelt oder aufgestrichen wird, dass es mit Abstand zu der Schweißtnaht (16) angeordnet ist.

2. Verfahren nach Anspruch 1, **dadurch gekennzeichnet dass** das Trägermaterial (22) so auf die Folienlage (11, 12) aufgebracht wird, dass es abziehbar an der Folienlage (11, 12) haftet.

3. Verfahren nach Anspruch oder 2, **dadurch gekennzeichnet, dass** das Trägermaterial (22) in demjenigen Endbereich des Transportbeutels angeordnet wird, der einer Einführöffnung (19), durch die der Innenraum (26) des Transportbeutels (10) zugänglich ist, abgewandt ist.

4. Verfahren nach einem der Ansprüche 1 bis 3, **dadurch gekennzeichnet, dass** als das Trägermaterial (22) ein aushärtbarer Kunststoff und/oder Klebstoff verwendet wird.

5. Verfahren nach einem der Ansprüche 1 bis 4, **dadurch gekennzeichnet, dass** Absorberpartikel (23) verwendet werden, die zumindest überwiegend aus zumindest einem superabsorbierenden Polymer (SAP) bestehen.

6. Verfahren nach einem der Ansprüche 1 bis 5, **dadurch gekennzeichnet, dass** die Absorberpartikel (23) in einem Anteil von ≥ 30mas% in dem Trägermaterial (22) vorhanden sind.

7. Transportbeutel (10) zum Transportieren von Flüssigkeitsproben, insbesondere Blut-, Urin-, Gewebeproben oder dergleichen, mit einer ersten Folienlage (11) und einer zweiten Folienlage (12), die jeweils aus Kunststoff bestehen und unter Bildung eines zwischen den Folienlagen (11, 12) ausgebildeten Innenraums (20) aufeinanderliegend angeordnet und unter Bildung einer Schweißnaht (16) miteinander verschweißt sind, wobei im Innenraum (20) eine Absorbereinrichtung (21) zum Aufnehmen von Flüssigkeit auf der dem Innenraum (20) zugewandten Innenseite (11a) zumindest einer der Folienlagen (11, 12) gehalten ist, **dadurch gekennzeichnet, dass** die Absorbervorrichtung (21) Absorberpartikel (23) aufweist, die an und/oder in einem Trägermaterial (22) angeordnet sind, dass das Trägermaterial (22) ein ausgehärteter Kunststoff und/oder Klebestoff ist, der auf die zumindest eine Folienlage (11, 12) aufgesprüht oder aufgespritzt oder aufgedruckt oder aufgerakelt oder aufgestrichen ist und in den die Absorberpartikel (23) eingemischt und/oder eingebettet sind, und dass das Trägermaterial (22) mit Abstand zu der Schweißnaht (16) angeordnet ist.

8. Transportbeutel nach Anspruch 7, **dadurch gekennzeichnet, dass** das Trägermaterial (22) abziehbar an der Folienlage (11, 12) haftet.

9. Transportbeutel nach Anspruch 7 oder 8, **dadurch gekennzeichnet, dass** das Trägermaterial (22) in demjenigen Endbereich des Transportbeutels angeordnet ist, der einer Einführöffnung (19), durch die der Innenraum (20) des Transportbeutels (10) zugänglich ist, abgewandt ist.

10. Transportbeutel nach einem der Ansprüche 7 bis 9, **dadurch gekennzeichnet, dass** die Absorberpartikel (23) überwiegend aus zumindest einem superabsorbierenden Polymer (SAP) bestehen.

11. Transportbeutel nach einem der Ansprüche 7 bis 10, **dadurch gekennzeichnet, dass** die Absorberpartikel (23) in einem Anteil von ≥ 30mas% in dem Trägermaterial (22) vorhanden sind.

## Claims

1. Method for producing a transport bag for transporting liquid samples, in particular blood, urine, tissue samples, or the like, wherein two film layers (11, 12) made of plastic are placed one on top of the other and fused to one another, forming a weld seam (16), in such a manner that an interior space (20) is formed between the film layers (11, 12), wherein a liquid or viscous carrier material (22) into which absorber particles (23) are or will be mixed is applied to at least one of the film layers (11, 12) before the film layers (11, 12) are placed one on top of the other, and that the carrier material (22) with the absorber particles (23) is subsequently dried and/or cooled, wherein the carrier material (22) is sprayed or spattered or printed or knife coated or brushed on in such a manner that it is arranged at a distance from the weld seam (16).

2. Method according to claim 1, **characterized in that** the carrier material (22) is applied to the film layer (11, 12) in such a manner that it adheres peelably to the film layer (11, 12).

3. Method according to claim or 2, **characterized in that** the carrier material (22) is arranged in the end region of the transport bag that faces away from an insertion opening (19) through which the interior space (26) of the transport bag (10) can be accessed.

4. Method according to one of claims 1 to 3, **characterized in that** a curable plastic and/or adhesive is used as the carrier material (22).

5. Method according to one of claims 1 to 4, **characterized in that** absorber particles (23) are used that consist at least predominantly of at least one superabsorbent polymer (SAP).

6. Method according to one of claims 1 to 5, **characterized in that** the absorber particles (23) are present in a proportion of ≥ 30 mas% in the carrier material (22).

7. Transport bag (10) for transporting liquid samples, in particular blood, urine, tissue samples, or the like, having a first film layer (11) and a second film layer (12) that are each made of plastic and are arranged one on top of the other, forming an interior space (20) between the film layers (11, 12), and are fused to one another, forming a weld seam (16), wherein, in the interior space (20), an absorption device (21) for accommodating liquid is held on the inner side (11a) of at least one of the film layers (11, 12) facing the interior space (20), **characterized in that** the absorption device (21) has absorber particles (23) that are arranged on and/or in a carrier material (22), **in that** the carrier material (22) is a cured plastic and/or adhesive that is sprayed or spattered or printed or knife coated or brushed onto the at least one film layer (11, 12) and in which the absorber particles (23) are mixed and/or embedded, and **in that** the carrier material (22) is arranged at a distance from the weld seam (16).

8. Transport bag according to claim 7, **characterized in that** the carrier material (22) adheres peelably to the film layer (11, 12).

9. Transport bag according to claim 7 or 8, **characterized in that** the carrier material (22) is arranged in the end region of the transport bag that faces away from an insertion opening (19) through which the interior space (26) of the transport bag (10) can be accessed.

10. Transport bag according to one of claims 7 to 9, **characterized in that** the absorber particles (23) consist predominantly of at least one superabsorbent polymer (SAP).

11. Transport bag according to one of claims 7 to 10, **characterized in that** the absorber particles (23) are present in a proportion of ≥ 30 mas% in the carrier material (22).

## Revendications

1. Procédé de fabrication d'un sachet de transport pour le transport d'échantillons liquides, en particulier d'échantillons de sang, d'urine, de tissus ou similaires, dans lequel deux couches de film (11, 12) en matière plastique sont posées l'une sur l'autre et soudées l'une à l'autre en formant un cordon de soudure (16) de sorte qu'un espace intérieur (20) est formé entre les couches de film (11, 12), dans lequel, avant la pose des couches de film (11, 12) l'une sur l'autre, un matériau de support (22) liquide ou visqueux est appliqué sur au moins l'une des couches de film (11, 12), matériau de support dans lequel des particules absorbantes (23) sont mélangées, et de sorte que le matériau de support (22) comportant les particules absorbantes (23) est ensuite séché et/ou refroidi, dans lequel le matériau de support (22) est pulvérisé ou projeté ou imprimé ou raclé ou étalé de sorte qu'il est disposé à distance du cordon de soudure (16).

2. Procédé selon la revendication 1, **caractérisé en ce que** le matériau de support (22) est appliqué sur la couche de film (11, 12) de sorte qu'il adhère de manière décollable à la couche de film (11, 12).

3. Procédé selon la revendication ou 2, **caractérisé en ce que** le matériau de support (22) est disposé dans la zone d'extrémité du sachet de transport qui est opposée à une ouverture d'introduction (19) par laquelle l'espace intérieur (26) du sachet de transport (10) est accessible.

4. Procédé selon l'une des revendications 1 à 3, **caractérisé en ce qu'**une matière plastique durcissable et/ou un adhésif sont utilisés comme matériau de support (22).

5. Procédé selon l'une des revendications 1 à 4, **caractérisé en ce que** des particules absorbantes (23) sont utilisées, lesquelles sont constituées au moins majoritairement d'au moins un polymère superabsorbant (SAP).

6. Procédé selon l'une des revendications 1 à 5, **caractérisé en ce que** les particules absorbantes (23) sont présentes dans une proportion ≥ 30 mas% dans le matériau de support (22).

7. Sachet de transport (10) pour le transport d'échantillons liquides, en particulier d'échantillons de sang, d'urine, de tissus ou similaires, comportant une première couche de film (11) et une seconde couche de film (12), lesquelles sont respectivement constituées de matière plastique et sont disposées l'une sur l'autre en formant un espace intérieur (20) réalisé entre les couches de film (11, 12) et sont soudées l'une à l'autre en formant un cordon de soudure (16), dans lequel, dans l'espace intérieur (20), un appareil d'absorption (21) permettant la réception de liquide est maintenu sur le côté intérieur (11a) d'au moins l'une des couches de film (11, 12) orienté vers l'espace intérieur (20), **caractérisé en ce que** le dispositif d'absorption (21) présente des particules absorbantes (23) disposées sur et/ou dans un matériau de support (22), **en ce que** le matériau de support (22) est une matière plastique durcie et/ou un adhésif, lequel matériau de support est pulvérisé ou projeté ou imprimé ou raclé ou étalé sur l'au moins une couche de film (11, 12) et dans lequel les particules absorbantes (23) sont mélangées et/ou intégrées, **et en ce que** le matériau de support (22) est disposé à distance du cordon de soudure (16).

8. Sachet de transport selon la revendication 7, **caractérisé en ce que** le matériau de support (22) adhère de manière décollable à la couche de film (11, 12).

9. Sachet de transport selon la revendication 7 ou 8, **caractérisé en ce que** le matériau de support (22) est disposé dans la zone d'extrémité du sachet de transport qui est opposée à une ouverture d'introduction (19) par laquelle l'espace intérieur (20) du sachet de transport (10) est accessible.

10. Sachet de transport selon l'une des revendications 7 à 9,
**caractérisé en ce que** les particules absorbantes (23) sont majoritairement constituées d'au moins un polymère superabsorbant (SAP).

11. Sachet de transport selon l'une des revendications 7 à 10,
**caractérisé en ce que** les particules absorbantes (23) sont présentes dans une proportion ≥ 30 mas% dans le matériau de support (22).
